# EUROPEAN PATENT APPLICATION

(11) **EP 1 442 720 A1**
(43) Date of publication of application: **04.08.2004**
(21) Application number: 04000950.8
(22) Date of filing: 19.01.2004
(51) Int. Cl.: A61B 19/00, A61B 17/00, A61B 17/10

(54) **Apparatus for the maneuvering of flexible catheters in the human cardiovascular system**

(30) Priority: 31.01.2003 IT BO20030040; 03.12.2003 IT BO20030731
(71) Applicant: Tre Esse Progettazione Biomedica S.r.l, 40138 Bologna (IT)
(72) Inventor: Plicchi, Gianni, 40136 Bologna (IT); Marcelli, Emanuela, 62100 Macerata (IT)
(74) Representative: Porsia, Dino, Dr.

(57) **Abstract**

A remotely controllable robotic apparatus for the maneuvering of flexible catheters in the human cardiovascular system comprises means in the form of an arm (2) for the positioning, aiming and correct orientation with respect to the patient's body of a device (R) which supports at least one portion of the catheter (C) and which comprises remotely controllable actuators for transmitting to the said catheter at least a longitudinal movement of advance or withdrawal and/or a rightward or leftward rotary movement about its longitudinal axis, these actuators consisting of sets of opposing wheels or rollers parallel to each other, or equivalent means such as belts, connected to remotely controllable rotation means and positioned in such a way as to transmit the aforesaid movements to the catheter. The apparatus comprises a pair of rollers (W1, W2) parallel to each other and orthogonal to the catheter, connected wholly or partially to means of rotation (Z1) in both directions, to produce the longitudinal advance or withdrawal (Z10) of the said catheter, and connected to means for transmitting an axial movement (Z2) in one direction or in the opposite direction to at least one of the said rollers, in such a way as to cause the rotation (Z20) by rolling of the said catheter between the rollers, so that the catheter rotates about its own axis either to the right or to the left.

## Description

The insertion and maneuvering in the human body of flexible catheters for diagnostic and/or therapeutic purposes, for example in order to perform angiographic examination of the arteries, coronography, angioplastic procedures, procedures for measuring electrophysiological parameters, ablation of arrhythmogenic regions in the right- or left-hand cardiac chambers, and for the permanent implantation of electrocatheters for stimulation and/or defibrillation, is currently carried out manually by an operator who, after opening the access path for the catheter, introduces the latter into the said path with single or combined movements of advance, withdrawal and rightward or leftward rotation. Since the catheter is in direct contact with the blood, it is currently impossible to provide a direct image of the intravascular or intracardiac regions in which it is located, using the remote visual endoscopic and monitoring methods based on video cameras and optical fibres which are applied at present in operations on the urological, pulmonary and gastroenterological systems and generally in minimally invasive surgery. Consequently, in order to guide the catheter correctly in the insertion stage, the operator must rely on his own skill and sensitivity in detecting any impediments to the advance of the catheter, aided by indirect imaging systems such as X-ray fluoroscopy or ultrasound, which show the positions progressively assumed by the inserted portion of catheter in the human body. X-ray fluoroscopy is the most widely used method at present. The implantation of a catheter may take several minutes, or even hours in some cases where complex procedures are concerned, with possible damage to the operator subjected to the ionizing radiation of the imaging system, even if the operator uses all the known radiation protection systems. It should also be borne in mind that an operator can be required to carry out a number of successive operations, separated by short time intervals, causing an accumulation of physical stress and an accumulation of absorbed radiation, with a consequent increase of the probability of occurrence of biological damage to the operator's body.

The physical factors affecting the absorption of ionising radiation in organic material are time, distance and shielding. As the period of exposure to ionizing radiation decreases, the dose of such radiation absorbed by the body also decreases. The absorption of radiation also falls significantly with an increase in the distance from the radiation source and with the interposition of shielding based on lead sheet and/or other types of shielding.

One possible innovative solution for significantly reducing operators' exposure to X-rays during the procedure of maneuvering catheters within the body, for diagnostic and/or therapeutic purposes, consists in the introduction of robotic systems for executing this procedure, controlled by the operator from a suitably shielded remote position, as in the case of systems for the remote handling of radioactive materials.

The applications of robotics, initially limited to industrial environments, are now seen in many different fields. In addition to the use of robotic manipulators and mobile robots in space and more generally in high-technology environments, the principles of robotics are commonly applied in public services, including medicine.

At present, the principal applications of robotics in the medical and surgical fields are found in:
- surgical operations (robots for microsurgery, endoscopy, orthopaedic surgery and minimally invasive surgery);
- examination and monitoring (robots for sampling and tissue transport);
- basic research (robots for cellular surgery and simulation);
- training (robots for training in anaesthesia, emergency medicine or for surgical training and simulation);
- assistance to patients (robots for assistance to patients (robot nurses), robots for assistance to the disabled);

Recent applications relate to remote manipulation of flexible endoscopes for the investigation of the gastrointestinal and pulmonary system.

A robot is a programmable automatic electromechanical device, initially developed for industrial applications, capable of executing predetermined operating cycles more rapidly, accurately and economically than a human operator, and operating in positions or in conditions that would be hazardous for humans. Robots are ideal for procedures which are monotonous and repetitive and which would soon cause fatigue in the operator or which would be hazardous for humans.

The introduction of robotics in surgery provides significant advantages, since the monitoring of the surgical operation is better than that obtainable with conventional endoscopic instruments, and also permits remote manipulation, in other words the surgeon/operator can be physically remote/distant from the operating theatre.

Up to the present time, there has been no description of a robotic device which would permit the remote manipulation of endocardiovascular catheters for therapeutic and/or diagnostic purposes, with consequent advantages of mitigating the physical stress on the operator and enabling him to move and guide the catheter from a remote and suitably shielded position. Such a device could be applied in all invasive cardiology procedures requiring the insertion and manipulation of catheters inside the cardiovascular system, such as the performance of angiographic arterial examination, coronography, angioplasty procedures with or without the positioning of stents, electrophysiological parameter measurement procedures, ablation of arrhythmogenic regions in the right- and left-hand cardiac chambers, permanent implantation of stimulation and/or defibrillation electrodes and intervention for the introduction of genetic material into the cardiovascular system.

To resolve these and other problems of the known art, the invention proposes an apparatus of the robotic type, which is remotely controllable by an operator who is sheltered in a shielded environment, and which can provide precise maneuvering of a flexible catheter in the human cardiovascular system, with the aforesaid advantages.

The apparatus according to the invention comprises a positioning and orientation system consisting, for example, of an arm which is articulated, jointed or movable on orthogonal axes, allowing precise positioning of the said apparatus with respect to the patient, who in turn is suitably immobilized and positioned in relation to the said apparatus. The positioning arm can remain fixed during the intervention or can if necessary be movable and adjustable. In an initial stage, using a widely standardized surgical procedure, the operator prepares the access path for the catheter into the human body and manually executes the first stage of introduction of the said catheter, making incisions in the skin, vein or artery and providing suitable movable ligature means for controlling haemostasis. The action of these means for controlling haemostasis can also be remotely monitored and controlled. At the distal end of the said positioning arm there is mounted a box containing the motors, actuators, power supply batteries and the electronic control card or cards, and a terminal, preferably disposable, is fixed removably to this box and has groups of wheels and/or belts controlled by a first drive unit, for the longitudinal movement of the catheter which can thus be made to advance or withdraw, and by a second drive unit for the rightward or leftward rotation of the catheter about its longitudinal axis. If the catheter which is used contains within it an axially movable stylet for controlling the shape and stiffness of the said catheter, the box can contain a third drive unit which drives means for moving this stylet longitudinally forwards and backwards. On the other hand, if the catheter is of the steerable type, the apparatus can comprise means driven by remotely controllable actuators, also housed in the drive box, for guiding the said catheter, in order to transmit to the tip and body of the said catheter the bends and/or rotations necessary to reach the desired position within the cardiovascular system. The drive units and the actuators employed make use of small motors and/or other systems with encoders, which can be operated precisely by remote control, for example stepping motors or other motors or actuators with electronic speed and phase control. Means can be provided to measure and display the force exerted by the motors, in such a way as to transmit this information to the operator who is thus made directly aware of the resistance of the catheter to the various movements imparted to it, as he would be when manipulating the said catheter directly. This information can be used as comparison terms by the operator or if necessary for automating part of the operating cycle of the apparatus, for example in order to stop the current operation automatically when predetermined threshold values are exceeded for a predetermined time. Suitable safety means will in any case be provided to make the remotely controlled movement of the catheter safe and reliable, and to enable it to be carried out according to predetermined procedures. The operator, being shielded from the ionising radiation, will remotely control the robotic apparatus in question, to advance or withdraw the catheter and/or to rotate it rightwards or leftwards about its axis, with the advantage that he will also be able to carry out the movements in a composite and essentially continuous way. At the control station, the operator will be provided with any necessary means for controlling the haemostasis of the path into which the catheter is inserted and any necessary means for measuring the resistance of the catheter to movement, as well as the commonly used means of displaying the investigation carried out by X-ray fluoroscopy or other means of examination, making him aware of the progressive positioning of the catheter in the patient's body and enabling him to react in real time to any anomalies which are detected. Clearly, the operator will also have the use of a video camera and a monitor for remotely viewing the region of the patient's body in which the intervention is taking place, to provide him with all the information which the said operator would have if he were close to the patient, while, as stated above, he remains sheltered in a remote environment and is shielded from the source of ionizing radiation emitted by the display system used on the patient.

Further characteristics of the invention, and the advantages derived therefrom, will be made clearer by the following description of some preferred embodiments thereof, illustrated purely by way of example and without restrictive intent in the figures of the attached sheets of drawings, in which:
- Figure 1 is a lateral elevation of the robotic apparatus which can be positioned and orientated for example with respect to the bed on which the patient is laid, and shows a block diagram of all the components which enable the said apparatus to operate and be used remotely;
- Figure 2 is a lateral view in partial section of a first embodiment of the apparatus;
- Figure 2a shows details of the solution of Figure 2, in a view taken through the section lines II-II;
- Figure 3 is a lateral view in partial section of a second embodiment of the apparatus;
- Figure 4 shows other details of the solution of Figure 3, in a view taken through the section lines IV-IV;
- Figure 5 is a perspective view of a pair of drive rollers according to a third embodiment of the apparatus according to the invention;
- Figure 6 is a front view, in partial section, of one of the drive units of the apparatus according to the solution of Figure 5;
- Figure 7 is a perspective view of the robotic apparatus according to the solution shown in Figures 5 and 6.

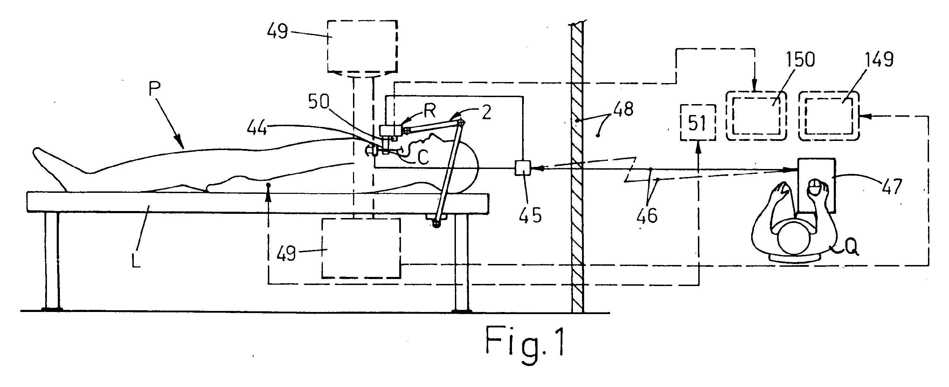

Figure 1 shows that the robotic apparatus R according to the invention comprises a box 1 (see below) which for example can be fixed to the bed L on which the patient P is immobilized and correctly positioned, for example by means of a connecting arm 2 articulated and/or jointed about a plurality of axes which can be securely locked, the whole being arranged in such a way that the said apparatus R can be positioned close to the pathway formed in the patient for the introduction into it of the catheter, with the necessary orientation and alignment for the subsequent correct maneuvering of the said catheter. Once positioned by the operator, the connecting arm 2 remains fixed. However, it should be understood that other suitable systems for supporting and orientating the box 1 can be used in place of the arm 2, including systems which are not connected to the bed L and which are therefore autonomous, and systems articulated and/or sliding on orthogonal axes, with independent and remotely controllable drive means, such as a true robotized movement system, movable about a plurality of axes, the whole being designed in a way which can be understood and easily implemented by persons skilled in the art.

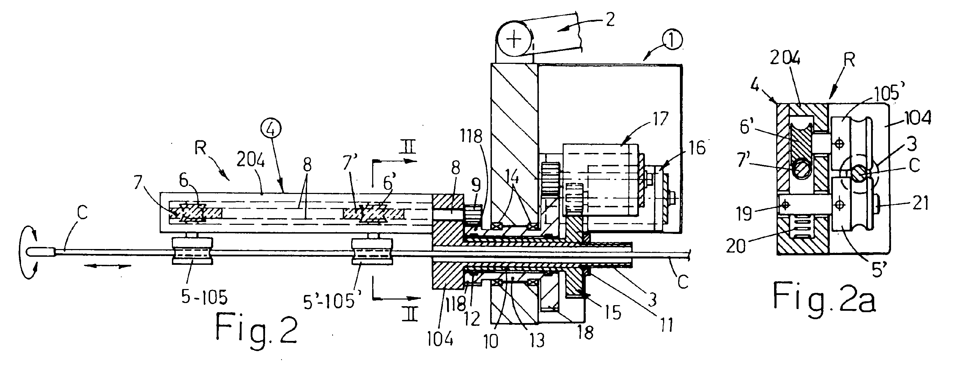

In a first embodiment, shown in Figures 2 and 2a, the apparatus R can comprise a set of rollers which grip the catheter and which can be given a rotary movement about their axes to move the said catheter longitudinally forwards or backwards, and which can be made to rotate or revolve about the axis of the catheter to transmit to it a rightward or leftward rotation about its axis. Figure 2 shows that the catheter C passes through a hub 3 perpendicularly integral with the small end wall 104 of an L-shaped support 4, whose longitudinal wall 204 has its longitudinal axis parallel to the axis of the spindle 3 and carries laterally, in an orthogonal arrangement, at least one unit, for example a pair of parallel rollers 5, 105 between which the catheter passes tangentially and is gripped by a sufficiently extended contact with the curved tread, having a high coefficient of friction, of these components, as shown in Figure 2a. The region of the rollers which interacts with the catheter is aligned with the longitudinal axis of the hub 3, in such a way that the catheter leaving this hub is then correctly picked up and guided by the said rollers without being subjected to undesirable bending. Preferably, the support 4 is provided with a pair of parallel rollers 5, 105 a short way away from the distal end of the wall 204 and with a further pair of parallel rollers 5', 105' a short way away from the wall 104, in such a way that these rollers act on the catheter as soon as it leaves the hub 3 and the other rollers act on the catheter shortly before it leaves the apparatus and enters the patient's body. The driving rollers 105, 105' have small helical wheels 6, 6' keyed on their shafts, these wheels engaging with screws 7, 7' keyed to or integral with a common shaft 8 supported rotatably by the wall 204 of the support 4 and carrying at its end a pinion 9. The aforesaid hub 3 is inserted axially into a hollow shaft 10 and is keyed and locked axially in this by means of couplings which are not illustrated, for example by means of a nut 11 mounted on a threaded terminal portion of the said hub 3. A quick-release coupling, of the magnetic type for example, can be used as an alternative to the nut 11. The hollow shaft 10 is in turn mounted by means of bushes or bearings 12 in another, outer hollow shaft 13 which in turn is supported rotatably, by means of bushes or bearings 14, by the base of the box 1, and which is positioned perpendicularly to this base. The shaft 10 is connected by means of gearing 15 to a drive unit 16 flanged on to the inner face of the base of the box 1, together with a drive unit 17 which by means of gearing 18, 118 transmits the rotation to the outer hollow shaft 13 and then to the pinion 9 of the operating part of the device R. The drive units 16 and 17 comprise reversible electric motors suitable for remote electronic speed and phase control, for example stepping, brushless or other suitable motors. Clearly, the rotation of the drive unit 17 causes the rotation of the rollers 5, 105, 5', 105' about their axes and therefore the forward or backward longitudinal movement of the catheter, while the rotation of the drive unit 16 causes the rotation or revolution of the said drive rollers about the axis of the catheter, with rightward or leftward rotation of the said catheter about its own axis. With this solution, the rotary movement transmitted to the catheter through the drive unit 16 causes, by reaction with the gearing 118, 9, a simultaneous longitudinal movement of the said catheter which can be eliminated or corrected by the simultaneous activation of the drive unit 17 in the appropriate direction. Figure 2 shows how both drive units 16 and 17 can be located on the same inner face of the base of the box 1 which additionally acts as a shield for these components which will be in different angular positions with respect to the corresponding hollow shafts 10 and 13. The box 1 can also house electronic cards for the remote control of the two drive units, a rechargeable battery for supplying electrical power to the system, and all the other necessary means.

In the initial stage of preparation of the apparatus for intervention on the patient, the catheter leaving the hub 3 must be clear of the drive rollers 5, 5', 105, 105', so that it can be freely manipulated by the operator who must introduce it into the path opened in the patient's body. When the catheter has been introduced, and after the apparatus R has been positioned as closely as possible to the patient and with the correct orientation, the operator must be able to move the drive rollers apart from each other to position the catheter tangentially between them. To resolve all these problems, the axes of the driven rollers 5, 5' are, for example, mounted on the support wall 204 the possibility of articulation on a pivot 19 and are pushed against the driven rollers by a spring 20 (Fig. 2a). A short portion of the journal of each idle shaft projects, for example, frontally from these rollers, as indicated for example by 21, in such a way that the driven rollers can be raised with a finger in opposition to the spring 13 and moved away from the driving rollers by the amount required to position the catheter C between the said rollers and to remove it from them. It should be understood that a mechanism with simplified centralized control can be provided, also on the base of the box 1, to facilitate this maneuver, the whole being arranged in a way which can be understood and easily implemented by persons skilled in the art.

Clearly, all the parts 3, 4 with the attached catheter movement rollers and the corresponding drive transmission 6-9 can be produced economically in disposable form, since these parts may be contaminated with organic material during the operation of the apparatus.

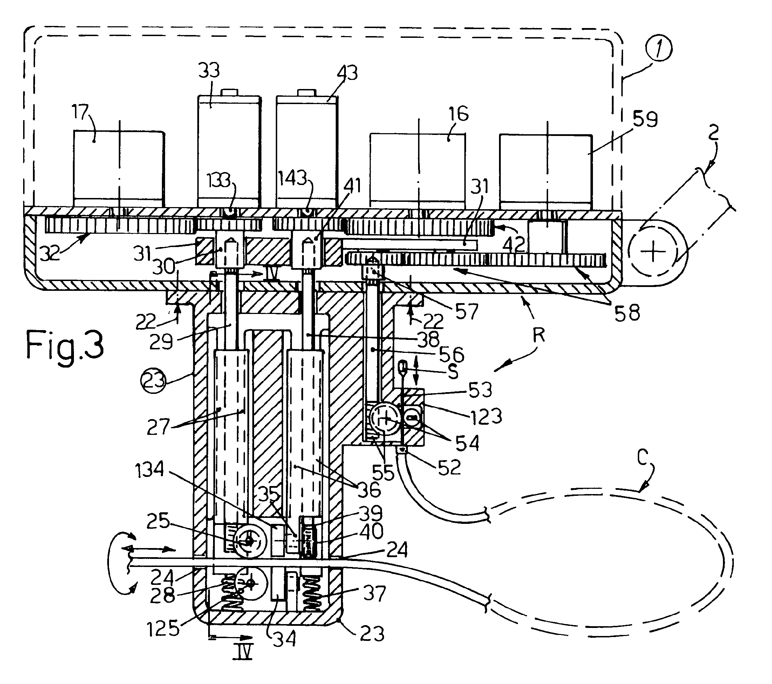

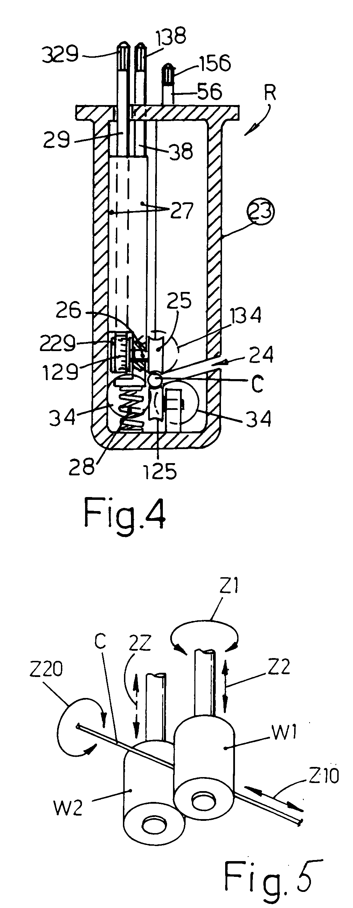

Figures 3 and 4 show a preferred embodiment of the device R in question, in which at least one specific set of rollers is provided for the longitudinal drive of the catheter, and a different set of rollers is provided for the express purpose of rotating the said catheter about its axis. In Figures 3 and 4, the number 1 indicates the box associated with the positioning arm 2, while 23 indicates the disposable part of the apparatus which is fixed removably to the box 1 by quick-release fasteners, shown schematically by the arrows 22. The part 23 comprises a parallelepipedal and internally hollow body, with rounded edges and corners, whose lower end, which is to be positioned close to the patient's body, is provided with a transverse oblique blind groove or channel 24, into which is inserted the catheter C which passes longitudinally through this channel. The channel 24 is inclined in such a way that the catheter is impelled by gravity into the innermost part of the said channel. The portion of catheter passing through the channel 24 bears tangentially on at least one orthogonal roller 125, with a curved tread profile, mounted in a static rotatable way in the body 23, and a second roller 25 is provided above and parallel to this roller, the shaft 26 of the second roller being supported rotatably by a sliding block 27 guided vertically in the body 23 and pushed upwards by a spring 28, in such a way that the roller 25 does not normally interact with the catheter. A vertical shaft 29, whose lower end carries a worm gear 129 interacting with a helical gear 229 keyed on the axle 26 of the roller 25, is mounted rotatably on the sliding block 27. The upper end of the shaft 29 has a toothed coupling 329 for rapid and removable keying on a power take-off 30 guided axially in a support 31 of the box 1 and connected by means of gearing 32 to the drive unit 17 flanged in the said box 1, together with a solenoid 33 whose rod 133 interacts with the power take-off 30. When the rod 133 is raised, the drive roller 25 is positioned away from the catheter C which can thus be inserted into the guide channel 24 or removed therefrom. On the other hand, when the rod 133 is lowered in opposition to the spring 28, the sliding block 27 is lowered and the roller 25 interacts frictionally with the catheter whose underside bears on the driven roller 125, and the said catheter can be made to advance or withdraw by the action of the drive unit 17.

The portion of catheter which passes through the channel 24 also bears between and on a pair of rollers 34 which are parallel to each other and to the said catheter and are supported rotatably by the body 23. Above the rollers 34 there is provided, in a symmetrical and parallel arrangement, a third roller 134, whose shaft 35 is supported rotatably by a sliding block 36 movable vertically in the body 23, pushed upwards by a spring 37 and carrying a rotatable vertical shaft 38 which has a lower worm gear 39 interacting with a helical wheel 40 keyed on the axle of the roller 134. The upper end of the shaft 38 is provided with a toothed coupling 138 for rapid and removable keying to a power take-off 41 which is guided axially in the said support 31 of the box 1 and which is connected by means of gearing 42 to the drive unit 16 flanged in the said box 1, together with a solenoid 43 whose rod 143 interacts with the power take-off 41. When the rod 143 is raised, the roller 134 is raised from the catheter C which can thus be inserted into the guide channel 24 or removed therefrom. On the other hand, when the rod 143 is lowered by the corresponding solenoid, in opposition to the spring 37, the sliding block 36 is lowered and the roller 134 interacts frictionally with the catheter whose underside bears on the driven rollers 34, and the said catheter can be made to rotate about its own axis by the action of the drive unit 16, with rightward or leftward rotation.

The catheter C can contain a metal stylet, which has a portion, of precise length, normally projecting from the rear end of the said catheter. There is a known way of subjecting the stylet to axial movements, first of withdrawal and then of advance, with respect to the catheter during the insertion and maneuvering of the catheter in the patient's body, in such a way that the flexibility of the catheter tip is varied, to facilitate the advance of the said catheter into the patient's body. Figure 3 shows that the apparatus according to the invention can be provided with means for additionally executing the said longitudinal movement of the catheter stylet by remote control.

These means comprise, for example, a device 52 for removably, rotatably and rapidly fixing the rear end of the catheter C to an appendage 123 of the body 23 of the disposable part of the apparatus, in which appendage there is provided a vertical channel 53 into which can be inserted the free rear portion of the stylet S, which is gripped between a pair of parallel sprung rollers 54, with an opening control for positioning the stylet between them or removing it therefrom, the rollers being connected by means of gearing 55, of the helical gear and worm type for example, to a vertical shaft 56 supported rotatably by the body 23 and projecting above this body, in the same way as the other shafts 29 and 38 (Fig. 4). The shaft 56 is connected by its upper toothed coupling 156 to a power take-off 57 which is connected by the gearing 58 to a drive unit 59 which is also housed in the box 1 and is of the same type as the aforesaid units 16, 17. Clearly, by operating the unit 59 for rightward or leftward rotation, the pulling rollers 54 can be rotated in a controlled way in the desired direction and consequently the stylet S can be moved longitudinally as required. It should be understood that the longitudinal movement of the stylet can be achieved with means other than those described, and with remotely controllable actuators, of the linear instead of the rotary type.

Figure 1 shows that the apparatus also comprises a ligature device 44 for controlling haemostasis, this device having the function of supporting the end of the vessel opened by the operator for the insertion of the said catheter C, and of keeping it stationary and sealed on the catheter, while allowing the sliding and rotation of the said catheter. This device, which must be disposable, can be operated by suitable means for increasing or decreasing the pressure of the grip on the vessel, preferably remotely controllable, the whole being arranged in a way which can be understood and easily implemented by persons skilled in the art. The apparatus R and the ligature device 44 can be connected, for example, to an interface 45 which can advantageously be housed in the box 1 and which is connected via a wire or radio link 46 to a control unit 47 located in a remote position within a shielded room 48 from which the operator Q can activate and remotely control the operation of the various parts of the apparatus which physically maneuver the catheter C in the patient's body up to the desired point. At the control station, the operator will also be provided with the screen and controls 149 for the fluoroscopic viewing system 49 and may also be provided with a viewer 150 for observing through at least one video camera 50 the details of at least the region of the patient on which he is intervening. The video camera can be of limited size and can usefully be fixed to the box 1 of the apparatus in question. The operator Q will also be able to monitor, via a monitor 51 and/or other suitable means, the various relevant and significant physiological conditions of the patient. The remote control unit 47 can also usefully contain means for measuring parameters relating to the force used to advance and/or rotate the catheter during the remote manipulation with the apparatus, in such a way as to transmit to the operator an equivalent of the sensitivity which the said operator previously had in the direct manipulation of the catheter. These parameters can also be used for the automatic control of the operation of the apparatus, for example in order to stop the current operation and to reverse it if necessary if excessive force continuing beyond a specified time interval is detected. If the catheter has electrodes or other suitable means, it can be used actively for carrying out impedance measurements and/or other readings for the purpose of detecting any anomalies during the stage of insertion of the catheter.

The systems for the remote control of the described apparatus can comprise voice control systems.

If the catheter is of the steerable type, the said apparatus can comprise means with linear or rotary actuators, with encoders, also remotely controllable and located in the box 1, for acting on the rear guides of the catheter in order to transmit to the distal tip and to the body of the said catheter the bending and/or rotation required to reach the desired position inside the cardiovascular system.

In the solution shown in Figures 3 and 4, which is to be considered preferable because it allows the catheter to be disengaged rapidly from the remote manipulation system at any time, the movement of advance or withdrawal of the said catheter is provided by as many as five rollers. If it is considered that, in certain cases, it must be possible to impart the aforesaid movements of advance, withdrawal and rightward and leftward rotation both to the body of the catheter and also to any internal stylet or guide mandrel, the device would require the presence of a total of ten rollers, and would become significantly bulky and would have excessively high production costs for a disposable terminal. The solution described below with reference to Figures 5, 6 and 7 is designed to overcome this significant technical and economic problem on the basis of the following idea.

In Figure 5, W1 and W2 indicate two rollers, parallel to each other, made from suitable material (see below), and orthogonal to the catheter C, which slides and is gripped between these rollers. By contrast with the preceding solutions, in which the catheter drive rollers have outer lateral surfaces with grooved profiles, with the additional function of guiding the said catheter, each of the rollers W1 and W2 according to the new solution has a right-angled generatrix, since the catheter must also be able to revolve on the generatrices of these rollers, as stated previously. At least one of the rollers, for example W1, is connected to a source of rotation as indicated schematically by the arrow Z1, and at least one of the said rollers, for example the driven roller W2, is pressed elastically against the other, to provide a secure frictional clamping of the interposed catheter. As a result of the rotation of the roller W1 in the clockwise or anti-clockwise direction, as indicated by the arrow Z1, the catheter C moves longitudinally forwards or backwards as indicated by the arrow Z10. According to the solution in question, at least one of the two rollers, for example W1, is also connected to a source of axial movement in both directions, as indicated by the arrows Z2, in such a way that, as a result of this movement, the catheter C gripped between the rollers W1 and W2 is made to rotate rightwards or leftwards about its own axis, as indicated by the arrow Z20. There are clear advantages to be obtained from the use of a single pair of rollers, always engaged with the member C, to produce both the longitudinal advance and withdrawal movements of this member, and its rightward or leftward rotation, possibly simultaneously with the said longitudinal movement. If the axial movement Z2 is imparted to one of the rollers only, for example only to the roller W1 as suggested above, the catheter C is moved transversely and its position in space is changed. To avoid this outcome, it is preferable to have both rollers W1 and W2 connected to the said source of axial movement, so that they can make synchronized axial movements in opposite directions, as indicated by the arrows Z2 and 2Z.

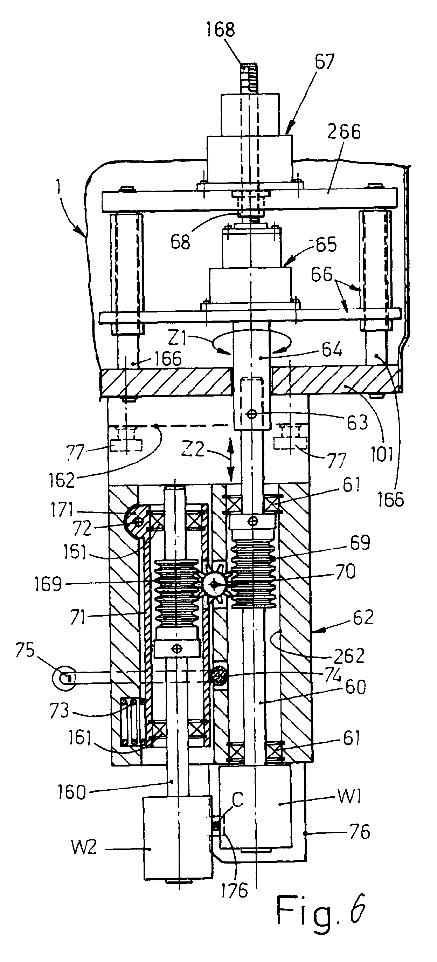

With reference to Figure 6, a description will now be given of a possible practical embodiment of the said system for the movement of the rollers W1 and W2. The roller W1 is keyed on the end of a shaft 60 mounted rotatably and with the possibility of axial movement, for example by means of bearings 61 of suitable plastic material, in a supporting body 62 from which the said shaft projects with an end portion opposite the end carrying the roller, for rapid and removable keying, by means of a pin 63 for example, to a hollow shaft 64 which projects from the base 101 of the box 1 containing the drive equipment of the robotic apparatus in question, the said support 62 being fixable rapidly and removably to this base by its own base piece 162, for example by means of screws 77. The hollow shaft 64 is connected to a geared motor unit 65 with an electric motor, of the stepping type for example, fixed on a sliding block 66 which slides while being guided parallel to the said shaft 64, on guides 166 fixed on the said base 101 and to an opposing base piece 266 on which is mounted a geared motor unit 67 with an electric motor, of the stepping or other type with electronic speed and phase control for example, which rotates a nut 68 interacting with a worm gear 168 integral with the sliding block 66 or with one of the components installed thereon, for example with the body of the unit 65 or of a link (not illustrated) which is fitted on top of this unit. Clearly, the activation of the unit 65 causes the rightward or leftward rotation Z1 of the roller W1, and the activation of the unit 67 causes the axial movement Z2 of the said roller W1. On the intermediate portion of the shaft 60, which slides in the cavity 262 of the body 62, there is keyed a cylindrical rack 69 which engages with a pinion 70 mounted freely rotatably in a suitable seat of the body 62, this component being described more fully below.

The roller W2 is keyed on the end of a shaft 160 which by means of suitable bearings 161 is mounted rotatably and with the possibility of axial movement in a bush 71 which has, on its end opposite that facing the said roller, an appendage 171 pivoted transversely, at 72, inside the body 62 and pushed by a spring 73 in such a way that the roller W2 is pressed against the roller W1 to provide the necessary secure frictional grip of the catheter C located between the said rollers W1 and W2. On the portion of shaft 160 lying within the bush 71, there is keyed a cylindrical rack 169, identical to the rack 69, which engages with the pinion 70 which passes through a lateral aperture in the said bush 71. Clearly, the action of the unit 69, 70, 169 is such that any axial movement imparted to the roller W1 by the unit 67 results in a corresponding axial movement of the roller W2 through an equal distance and in the opposite direction.

An eccentric 74, which interacts with the bush 71 and which can be rotated by at least one external end lever 75, is mounted rotatably inside the body 62 and orthogonally to the shafts 60, 160. When necessary, the lever 75 can be rotated to bring the part of the eccentric 74 having the greatest eccentricity into contact with the bush 71 and then to move the roller W2 away from the roller W1 through a sufficient distance to enable the catheter C to be removed from the said rollers W1, W2 or to be inserted between them. Figure 6 also shows how the catheter C can be correctly held between the rollers W1 and W2 by guide loops 76 which are located upstream and/or downstream of the pair of rollers, are integral with the body 62 and are each provided with a slot 176 into which the catheter can be inserted after the roller W1 has been temporarily moved away from the roller W2.

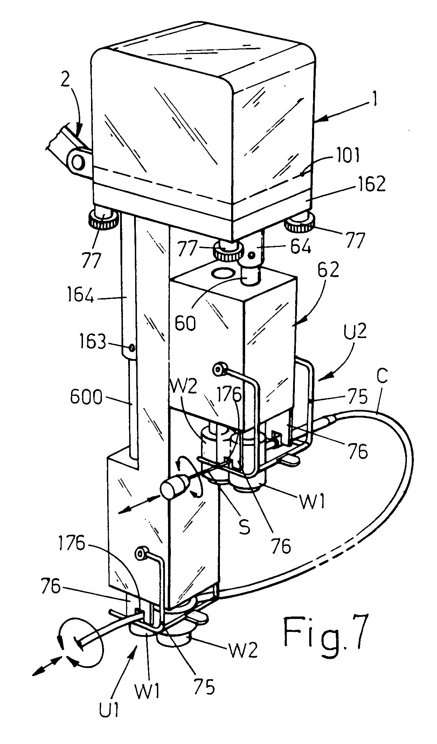

Figure 7 shows how the robotic apparatus according to the new solution has a first unit U1 and a second unit U2, positioned at different heights and with the pairs of rollers W1, W2 parallel to each other, incorporated in the disposable body 62 which is fixed removably to the base piece 101 of the box 1 which is associated with the support and positioning arms 2. The unit U1, which is in the lower position, has parallel rollers W1 and W2 with vertical axes, which interact with the body of the catheter and which are covered externally with a suitable elastomeric material, with appropriate characteristics of elasticity and yield. The unit U2, which is in the higher position, has a pair of parallel rollers W1 and W2, also with vertical axes, designed to interact with the guide stylet or mandrel S and made with external lateral surfaces consisting, for example, of satin stainless steel. The drive shafts 60 and 600 of the units U1 and U2 are designed to be keyed by the means 63 and 163 respectively to the corresponding motor and hollow shafts 64, 164 which project from the base 101 of the box. Finally, Figure 7 shows that, in order to facilitate the insertion of the parts C and S between the corresponding pairs of rollers W1, W2 and their removal therefrom, the loops 76 can alternatively have their guide slots 176 open in the direction of insertion or removal of the said parts C and S, and that each of these slots can be closed removably by removable means, for example by the parallel arms of a fork-shaped lever 75 fixed to both ends of the eccentric 74. If necessary, the guide loops 76 can be provided with parts for interaction by snap-fitting with the forked lever 75, to secure the latter in the position in which it closes the guide slots 176, the whole being arranged in a way which can be understood and easily implemented by those skilled in the art.

The pair of rollers which are parallel to each other have their axes orthogonal to the longitudinal axis of the catheter or of the guide mandrel, and generate the longitudinal movement of the said catheter or of the guide mandrel can not only be driven with a rotary movement in order to provide the said longitudinal movement of advance or withdrawal of the catheter, but can also be moved axially with respect to each other with a movement orthogonal to the axis of the catheter, to cause the rotation of the said catheter or of the guide mandrel by rolling about its own axis. Clearly, a simpler construction and a reduction of overall dimensions result from the fact that the body of the catheter and the guide mandrel can be driven in all the requisite ways by only four rollers which are constantly engaged with the corresponding components, instead of by ten rollers, as in the prior art, with six of these alternately disengaged to avoid impeding the longitudinal movement of the catheter or of the guide mandrel.

## Claims

1. Apparatus for the maneuvering of flexible catheters in the human cardiovascular system, **characterized in that** it comprises:
- Means (2), in the form of an arm for example, for positioning, aiming and correct orientation with respect to the patient of a device (R) for remote manipulation of the catheter;
- A device (R) which supports at least one portion of the catheter (C) and which comprises remotely controllable actuators, for transmitting to the said catheter at least a longitudinal movement of advance or withdrawal and/or a rightward or leftward rotary movement about its longitudinal axis;
- A control and monitoring unit (47) located in a remote position and protected in a shielded environment (48), by means of which the operator can remotely control and monitor the operation of the said device (R) which carries out the servo-controlled maneuvering of the catheter (C) in the patient's body;
- Means (46) for the operational remote connection of the said servo-controlled device (R) to the said control and monitoring unit (47).

2. Apparatus according to Claim 1, **characterized in that** it comprises means which, in response to a remote command from the said control unit (47), can execute a controlled forward or backward longitudinal movement and if necessary a rightward or leftward rotation of the metal stylet (S) normally present in the catheter (C), to facilitate and correct the advance of the said catheter in the patient's body.

3. Apparatus according to Claim 1, **characterized in that** it comprises a disposable device (44) for the ligature of the access vessel for the introduction of the catheter, which has the function of controlling the haemostasis and/or supporting the end of the said vessel into which the catheter (C) is to be inserted, while allowing the catheter to undergo the necessary sliding and rotary movements, this device (44) being supported by suitable means and being, for example, associated with means which enable the closing tension of the ligature (44) exerted on the vessel to be increased or reduced by remote control from the control and monitoring unit (47) located in the protected booth (48).

4. Apparatus according to Claim 1, in which all the parts intended to come into contact with the catheter (C) and with any corresponding stylet (S) are provided in a disposable component designed for rapid and removable mounting on a box (1 a) which contains all the actuators and means necessary for the operation of the said apparatus by remote control.

5. Apparatus according to Claim 4, **characterized in that** the catheter is of the steerable type, and the said apparatus comprises means driven by remotely controllable actuators, also housed in the box (1) containing the drive equipment, these actuators guiding the said catheter by transmitting to the tip and to the body of the said catheter the necessary bending and/or rotation to reach the desired position within the cardiovascular system.

6. Apparatus according to Claim 1, in which the catheter manipulation device (R) comprises units, for example pairs of rollers (5, 105, 5', 105') which are opposed, parallel to each other and orthogonal to the catheter, or equivalent means, such as belts, provided for example with treads with concave profiles, which enclose the catheter in a sufficiently well distributed way and which are made from material and in shapes such that they have a high coefficient of friction in relation to the said catheter, while treating the catheter as gently as possible, additional means being provided for transmitting to the said rollers a rotary movement about their axes for moving the catheter longitudinally forwards or backwards or for transmitting to the said rollers a movement of rotation or revolution about the longitudinal axis of the catheter, to rotate the said catheter about its axis to the right or to the left.

7. Apparatus according to Claim 6, in which some of the said rollers or belts which control a portion of the catheter are mounted statically and in a projecting way on a support wall (204) and are connected to a source of rotation, while the rollers or belts opposite the static ones are mounted so that they can oscillate on the said support wall, in opposition to elastic means (20), in order to grip the catheter by friction, to enable them to act on catheters of different diameters and to enable them to be moved away as necessary from the static rollers or belts whenever the catheter has to be inserted between the said movement rollers or belts or withdrawn therefrom, suitable means (21) being provided to facilitate this movement away.

8. Apparatus according to Claim 7, in which the wall (204) which carries the rollers or belts for supporting and moving the catheter has at its end a right-angled wall (104) which is fixed perpendicularly to the open end of a hub (3) through which the catheter passes longitudinally and is therefore aligned tangentially to the gripping surfaces of the said rollers or belts, this hub being inserted axially into a hollow rotation shaft (10) of the apparatus, supported rotatably by the base of the box (1), the hub being keyed and locked axially in this shaft by means of couplings and suitable means (11), in such a way that all the parts of the apparatus designed to come into contact with the catheter can be interchanged and are disposable, the said box (1) housing in a shielded position the necessary drive units (17, 16) for transmitting to the said rollers or belts both the rotary movement about their axes and the movement of rotation or revolution about the axis of the catheter.

9. Apparatus according to Claim 8, in which the hollow shaft (10) is connected by a drive transmission system (15) to a corresponding drive unit (16) which causes the rotation of the catheter in both directions and which is flanged on to the base of the box (1) of the apparatus, the said shaft (10) being mounted rotatably by means of bushes or bearings (12) in a second tubular shaft (13) supported rotatably by the said base of the box (1) by means of bushes or bearings (14), the pinion (118) which transmits the drive to the pinion (9) of the disposable part of the apparatus being keyed on one end of the said shaft (13), while a pinion (18) connected to the drive unit (17) for advancing and withdrawing the catheter is keyed on the other end of the said shaft (13).

10. Apparatus according to any one or more of the preceding claims, in which means are provided for enabling the catheter to be rapidly released at any time from the opposing manipulation rollers or belts, and also from the hollow shaft or shafts of the said apparatus, in such a way that it can be controlled freely and directly by the operator.

11. Apparatus according to Claim 1, in which the catheter manipulation device (R) comprises at least one specific set of rollers or belts (25, 125) for the longitudinal movement of the catheter, and a different set of rollers or belts (34, 134) expressly designed to rotate the said catheter about its longitudinal axis.

12. Apparatus according to Claim 11, **characterized in that** it comprises a box (1) connected to the positioning arm (2) and housing the drive equipment all means required for the remote operation of the said apparatus, this box having fixed to it, removably and with a downward extension, a parallelepipedal and internally hollow body (23), with rounded edges and corners, whose lower end, designed to be positioned close to the patient's body, is provided with a transverse oblique blind groove or channel (24), into which the catheter (C) is inserted, this channel being inclined in such a way that the catheter is impelled by gravity into the innermost part of the said channel, in which the rollers or belts for driving and/or rotating the catheter operate.

13. Apparatus according to Claim 12, in which the portion of catheter passing through the said channel (24) bears on at least one orthogonal roller (125), having a tread with a curved profile, mounted statically and rotatably in the body (23), a second roller (25) being provided above and parallel to this roller, the shaft (26) of the second roller being supported rotatably by a sliding block (27) guided vertically in the said body (23) and pushed upwards by an elastic means (28), in such a way that the said roller (25) does not normally interact with the catheter, a vertical shaft (29) being mounted rotatably on the sliding block (27) and having its lower end connected by gearing, of the worm gear (129) and helical gear (229) type for example, to the shaft (26) of the said roller (25), the upper end of the said shaft (29) having a toothed coupling (329) for rapid and removable keying to a power take-off (30) guided axially in a support (31) of the box (1) and being connected by means of gearing (32) to the drive unit (17) housed in the said box (1), together with an actuator (33) whose rod (133) interacts with the said power take-off (30), the whole being arranged in such a way that when the rod (133) is raised the drive roller (25) is located away from the catheter (C) which can thus be inserted into the guide channel (24) or removed therefrom, while when the said rod (133) is lowered in opposition to the spring (28) the driving roller (25) interacts frictionally with the catheter whose lower part bears on the driven roller (125) and the said catheter can be made to advance or withdraw by the action of the drive unit (17).

14. Apparatus according to Claim 12, in which the portion of catheter passing through the guide channel (24) also bears between and on a pair of rollers (34) which are parallel to each other and to the said catheter, and which are supported in a free-running way by the body (23), and a third driving roller (134) is provided in a symmetrical and parallel arrangement above the said rollers (34), the axle (35) of the third roller being supported rotatably by a sliding block (36) which is vertically movable in the said body (23) and is pushed upwards by an elastic means (37), and which carries a rotatable vertical shaft (38) which is mechanically connected by gearing (39, 40) in its lower part to the axle of the driving roller (134), the upper end of the shaft (38) being provided with a toothed coupling (138) for rapid and removable keying to a power take-off (41) which is guided axially in the said support (31) of the box (1) and which is connected by means of gearing (42) to the drive unit (16) housed in the said box (1) together with an actuator (43) whose rod (143) interacts with the power take-off (41), the whole being arranged in such a way that, when the said rod is raised, the driving roller (134) is raised from the catheter (C) which can thus be inserted into the guide channel (24) or removed therefrom, while when the said rod (143) is lowered in opposition to the spring (37) the driving roller (134) interacts frictionally with the catheter whose underside bears on the driven rollers (34) and the said catheter can be made to rotate about its axis by the action of the drive unit (16), with rightward or leftward rotation.

15. Apparatus according to Claim 12, **characterized in that** it comprises a device (52) for fixing the rear end of the catheter (C) removably, rotatably and rapidly to an appendage (123) of the body (23) of the disposable part of the apparatus, in which appendage there is provided a vertical channel (53) into which can be inserted the free rear portion of the stylet (S) of the catheter, which is gripped between a pair of parallel driving rollers (54) sprung with respect to each other and provided with a temporary opening device for the insertion of the stylet between them and its extraction therefrom.

16. Apparatus according to Claim 15, in which the driving rollers (54) of the stylet (S) are connected by gearing (55) to a vertical shaft (56) supported rotatably by the body (23) of the disposable part of the apparatus and having a portion projecting above this body and provided with a toothed coupling (156) which is connected to a power take-off (57) which in turn is connected by gearing (58) to a drive unit (59) which is also housed in the box (1), the whole being arranged in such a way that the rightward or leftward rotation of this unit causes the controlled rotation in both directions of the said driving rollers (54) which provide the necessary longitudinal movement of the stylet (S) of the catheter (C).

17. Apparatus according to Claim 9, in which the drive units of the rotating type (16, 17, 59) housed in the box (1) comprise reversible electric motors suitable for remote electronic speed and phase control, for example stepping or brushless motors or other suitable types, while any linear actuators located in the said box for executing other necessary movements are provided with encoders and/or are also equipped for remote control.

18. Apparatus according to Claim 1, in which the remote control unit (47) comprises safety means to ensure the safety of the operation of the remote maneuvering of the catheter in the patient's body, and to enable this operation to be completed by remote control, means being provided for this purpose and consisting for example of means for measuring the parameters relating to the force used to advance and/or rotate the catheter during the remote manipulation with the said apparatus, in such a way as to transmit to the operator an equivalent of the sensitivity which the said operator had previously in the direct manipulation of the catheter, provision being made for the possible additional use of these parameters for the automatic control of the operation of the apparatus, for example in order to stop the current operation and to reverse it if necessary when excess force continuing for more than a specified time interval is detected.

19. Apparatus according to Claim 1, in which means (149) are provided in the remote shielded station (48) from which the operator (Q) operates with the unit (47) for controlling and monitoring the said apparatus, for activating and for detecting remotely the operation of the viewing system (49), for example the X-ray fluoroscopy system, used on the patient to display the position assumed progressively by the catheter in the body of the said patient, and at least one screen (150) can be provided in the said station (48) with any necessary controls, connected to at least one video camera (50) placed near the patient (P), for example on the box (1), for observing at least the region of the said patient into which the catheter (C) is inserted.

20. Apparatus according to Claim 1, in which means (51) for measuring important physical parameters of the patient being treated are also provided in the remote shielded station (48) from which the operator (Q) operates with the remote control and monitoring unit (47) of the said apparatus.

21. Apparatus according to Claim 1, in which the said apparatus (R) and the ligature device (25) for controlling haemostasis can be connected to an interface system (26) suitable for communication over a distance with the control unit (47) located in the remote shielded room (48) in which the operator (Q) operates, by means of wire-based or wireless connection and/or communication systems (46).

22. Apparatus according to Claim 1, in which the systems for the remote control of the said apparatus can comprise voice control systems.

23. Apparatus according to Claim 1, in which the means, of the arm type (2) for example, for positioning the said apparatus with respect to the patient are such that they remain fixed when the said apparatus operates, or can be movable and adjustable by remote control.

24. Apparatus according to Claim 1, in which a rechargeable electrical battery for the autonomous operation, if necessary, of the said apparatus can be mounted in the box (1) or in another suitable position, in addition to the electronic card or cards.

25. Apparatus according to Claim 1, **characterized in that** it comprises a pair of rollers (W1, W2) parallel to each other and orthogonal to the catheter, connected wholly or partially to means (Z1) for rotation in both directions, for longitudinally advancing or withdrawing (Z10) the said catheter, and connected to means for transmitting to at least one of the said rollers an axial movement (Z2) in one direction or in the opposite direction, in such a way as to cause the rotation (Z20) of the said catheter by rolling between the rollers, so that the catheter rotates about its axis either to the right or to the left.

26. Apparatus according to Claim 25, **characterized in that** each of the rollers (W1, W2) has a cylindrical lateral surface, with a straight generatrix, in such a way that the catheter can be rotated by rolling on them.

27. Apparatus according to Claim 25, **characterized in that** the means which transmit to one of the rollers (W1) the axial movement which causes the rightward or leftward rotation of the catheter comprise means for transmitting an equal axial movement in the opposite direction to the other roller (W2), the whole being done in such a way that the catheter (C) rotates about its axis without transverse movement.

28. Apparatus according to Claim 1, **characterized in that**, if the catheter (C) has an internal metal stylet (S) acting as a guide mandrel, the said robotic apparatus is provided with two maneuvering units (U1, U2), each of which can transmit rightward or leftward rotary movements and/or longitudinal forward or backward movements to the catheter and to the guide mandrel.

29. Apparatus according to Claim 28, in which each maneuvering unit (U1, U2) carries a pair of parallel rollers (W1, W2) which grip the catheter (C) or the end of the said guide mandrel (S) in an orthogonal way and which can be driven selectively and independently, both in respect of the longitudinal movement and the rotation of the said catheter (C) and/or the guide mandrel (S).

30. Apparatus according to Claim 29, **characterized in that** the pair of rollers (W1, W2) for interacting with the catheter (C) are made or covered on their circumferences with a material suitable for the gentle frictional interaction with the said catheter, for example a suitable elastomeric material.

31. Apparatus according to Claim 29, **characterized in that** the pair of rollers (W1, W2) for interacting with the guide mandrel (S) are characterized on their circumferences by sleeves of material suitable for the gentle frictional interaction with the said guide mandrel, for example a steel sleeve with a satin external surface.

32. Apparatus according to Claim 27, **characterized in that** one of the rollers (W1) is the driving roller and is keyed on the end of a shaft (60) mounted rotatably and with the possibility of axial movement in a supporting body (62) from which the said shaft projects with an end portion opposite the end carrying the roller, for rapid and removable keying to a hollow shaft (64) which projects from the base (101) of the box (1) containing the drive equipment of the robotic apparatus in question, the said support (62) being fixable rapidly and removably to this base by its own base piece (162), the said hollow shaft (64) being connected to a first geared motor unit (65) with an electric motor, of the stepping type for example, fixed on a sliding block (66) which slides while being guided parallel to the said shaft (64), on guides (166) fixed on the said base (101) and to an opposing base piece (266) on which is mounted a second geared motor unit (67) with an electric motor, of the stepping or other type with electronic speed and phase control for example, which rotates a nut (68) interacting with a worm gear (168) integral with the sliding block (66) or with one of the components installed thereon, the whole being arranged in such a way that the activation of the said first drive unit (65) causes the rightward or leftward rotation (Z1) of the roller (W1), while the activation of the second drive unit (67) causes the axial movement (Z2) of the said roller (W1), a cylindrical rack (69) which engages with a pinion (70) mounted freely rotatably in a suitable seat of the supporting body (62) being keyed on the intermediate portion of the shaft (60) of the roller (W1).

33. Apparatus according to Claim 32, **characterized in that** the other roller (W2) is driven and is keyed on the end of a shaft (160) which is mounted rotatably and with the possibility of axial movement in a bush (71) which has, on its end opposite that facing the said roller, an appendage (171) pivoted transversely in the support body (62) and pushed by a spring (73) in such a way that the said roller (W2) is pressed against the roller (W1) to provide the necessary secure frictional grip of the catheter (C) or of the guide mandrel (S) located between the said rollers (W1, W2), a cylindrical rack (169), identical to the rack (69) of the shaft of the driving roller, which engages with the said pinion (70) which passes through a lateral aperture in the said bush (71), being keyed on the portion of shaft (160) of the driven roller lying within the said bush (71), the whole being arranged in such a way that, by the action of the said rack and pinion system (69, 70, 169) any axial movement imparted to the roller (W1) by the corresponding drive unit (67) results in a corresponding axial movement of the driven roller (W2) through an equal distance and in the opposite direction.

34. Apparatus according to Claim 33, **characterized in that** an eccentric (74) is mounted rotatably in the support body (62), orthogonally to the shafts (60, 160) of the rollers (W1, W2), and interacts with the said bush (71) and can be rotated by at least one external end lever (75) by means of which the part of the said eccentric (74) having the greatest eccentricity can be brought into contact with the said bush (71) when required to move the driven roller (W2) away from the driving roller (W1), through a distance sufficient to enable the catheter (C) or the guide mandrel (S) to be removed from the said rollers (W1, W2) or inserted between them.

35. Apparatus according to Claim 26, **characterized in that** there is provided, upstream and/or downstream of each pair of rollers (W1, W2), a loop (76) with a slot (176) in which the catheter (C) or the guide mandrel (S) slides in a guided way, to provide a correct initial positioning of these components with respect to the corresponding pair of movement rollers.

36. Apparatus according to Claim 35, **characterized in that** the slot (176) of the said guide loop or loops (76) opens orthogonally to the axes of the rollers (W1, W2) and preferably in the direction of the driven roller (W2).

37. Apparatus according to Claim 35, **characterized in that** the slot (176) of the said guide loop or loops (76) opens parallel to the axes of the rollers (W1, W2) and in the direction in which the catheter (C) or the guide mandrel (S) is inserted into it when these components are located between their pair of controlling rollers (W1, W2), removable means being provided for temporarily closing the open side of the said slot in order to retain therein the component to be guided (C, S).

38. Apparatus according to Claim 37, **characterized in that** the means for removably closing the slots (176) of the guide loops (76) can consist of the suitably shaped branches of the lever (75) which causes the rotation of the eccentric (74) which temporarily moves the sprung driven roller (W2) away from the driving roller (W1), for the stage of insertion of the catheter (C) or the guide mandrel (S) between these rollers.

39. Apparatus according to Claim 29, **characterized in that** the two units (U1, U2) for maneuvering the catheter (C) and the guide mandrel (S) are positioned on a single disposable supporting body (62) which can be fixed removably to the base piece (101) of the box (1) housing the drive equipment, which is associated with the positioning and supporting arms (2) of the said robotic apparatus, the said units being positioned at different heights and close to each other, the unit (U1) in the lower position being designed to interact by means of its rollers (W1, W2) with the body of the catheter (C), while the unit (U2) in the higher position interacts by means of its rollers (W1, W2) with the guide mandrel (S) of the said catheter.
